(19) Europäisches Patentamt

European Patent Office

Office européen des brevets

(11) Publication number: **0 359 366 B1**

# (12) EUROPEAN PATENT SPECIFICATION

(45) Date of publication of patent specification: **09.03.94**

(51) Int. Cl.⁵: **A61L 31/00**, A61L 2/08, C08G 63/00, A61L 33/00, C08L 69/00

(21) Application number: **89306832.0**

(22) Date of filing: **05.07.89**

The file contains technical information submitted after the application was filed and not included in this specification

(54) **Medical moldings and method for their preparation.**

(30) Priority: **06.09.88 JP 223042/88**
    **09.11.88 JP 283350/88**

(43) Date of publication of application:
    **21.03.90 Bulletin 90/12**

(45) Publication of the grant of the patent:
    **09.03.94 Bulletin 94/10**

(84) Designated Contracting States:
    **DE FR GB IT SE**

(56) References cited:
    **EP-A- 0 152 012**
    **EP-A- 0 152 825**
    **EP-A- 0 296 473**

(73) Proprietor: **MITSUBISHI KASEI CORPORATION**
    **5-2, Marunouchi 2-chome**
    **Chiyoda-ku**
    **Tokyo 100(JP)**

(72) Inventor: **Nakajima, Takashi**
    **B203 Ryoka-Ninomiya-Haitsu**
    **404-2 Ninomiya**
    **Nimomiya-machi Naka-gun**
    **Kanagawa-ken(JP)**
    Inventor: **Ohyama, Hajime**
    **205 Bira-Mizusawa**
    **229-1 Chigasaki**
    **Chigasaki-shi Kanagawa-ken(JP)**
    Inventor: **Koyashiki, Yoshiaki**
    **Mitsubishi-Kasei-Fujisawa-Ryo**
    **2-3-15 Hon-machi**
    **Fujisawa-shi Kanagawa-ken(JP)**

(74) Representative: **Woods, Geoffrey Corlett et al**
    **J.A. KEMP & CO.**
    **14 South Square**
    **Gray's Inn**
    **London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a medical molding, for example a disposable medical instrument, comprising a $\gamma$-ray resistant material which has little deterioration of colour or physical properties even when irradiation by $\gamma$-rays is carried out for sterilization, as well as to a $\gamma$-ray resistant material.

Aromatic polycarbonate resins have excellent mechanical and thermal properties, and have been used in various applications. In addition, since they have excellent transparency and sanitary properties and are tough and highly heat-resistant, they have been used for various medical instruments such as container-like packages for putting into and packaging injectors, surgical tools, intravenous injectors and operation tools, as well as for various medical parts for an artificial lung, artificial kidney, anesthetic absorption device, intravene connectors and accessories, medical equipment such as a blood centrifugator, surgical instruments and operation instruments. They have particularly been used for parts of artificial dializers, artificial livers, artificial hearts and lungs, injectors and serum separators, which are disposable.

These medical parts are usually completely sterilized, for example by a contacting-treatment with ethylene oxide, heat-treatment in an autoclave or irradiation-treatment with ionizing radiation such as $\gamma$-rays.

Among these, use of ethylene oxide gas is not preferred in view of the toxicity and instability of ethylene oxide, as well as circumferential problems according to disposal-treatment. Use of an autoclave involves the problems that the resin may be deteriorated upon high-temperature treatment, the energy cost is expensive and drying before use is necessary since moisture remains in the parts after treatment.

Accordingly, a sterilizing-treatment using ionizing radiation, which can enable a low-temperature treatment and which is relatively inexpensive, is used instead of the above-mentioned methods.

Examples of ionizing radiation are $\alpha$-rays, heavy electron-rays, proton-rays, $\beta$-rays, electron-rays, neutron-rays, $\gamma$-rays and X-rays, $\gamma$-rays being used preferably in industry.

However, when irradiated with $\gamma$-rays, an aromatic polycarbonate resin, which is optically transparent, turns yellow. Accordingly, there have been proposed a method of offsetting the yellow colour by, for example, previously mixing a blue colourant into the resin, blending in a boron compound [JP-A-61-215651 (1986)], and blending in a polyether or alkyl ether thereof [JP-A-62-135556 (1987)].

Aromatic polycarbonate resins obtained by the above methods have various problems in that the prevention of yellowing is not sufficient. If a yellowing inhibitor is used in such an amount as to be sufficient to develop the desired effect, it remarkably deteriorates the strength of the resin itself, which cannot then be used in view of the poor strength thereof.

Accordingly, it has not yet been possible to obtain aromatic polycarbonate resins having little yellowing and still possessing sufficient strength and toughness after a sterilizing-treatment by $\gamma$-rays, or medical parts using such resins.

EP-A-296,473, published between the priorty dates and the filing date of the present application, discloses a molding composition which comprises an aromatic polycarbonate resin and a stabilizing agent which may contain, inter alia, a halogen atom.

The present invention provides a medical molding, for example a disposable medical instrument, suitable for contacting with blood or a physiological solution, comprising a $\gamma$-ray resistant material comprising an aromatic polycarbonate resin, which $\gamma$-ray resistant material contains from 0.1 to 10.0% by weight of bromine atoms.

The present invention also provides a method of sterilizing a medical molding which comprises irradiating a molding as defined above with $\gamma$-rays.

The present invention additionally provides a $\gamma$-ray resistant material as defined above.

The $\gamma$-ray resistant material comprising the bromine-containing aromatic polycarbonate resin or bromine-containing aromatic polycarbonate resin further blended with propylene glycol shows less colour change and maintains its physical properties even after irradiation by the ionizing radiation.

The $\gamma$-ray resistant material may, for example, comprise an aromatic polycarbonate polymer, for example a copolymer, and an organic bromine-containing compound.

The $\gamma$-ray resistant material may also, for example comprise a brominated aromatic polycarbonate copolymer.

The $\gamma$-ray resistant material may additionally, for example, comprise an aromatic polycarbonate polymer, for example a copolymer, and a brominated aromatic polycarbonate copolymer.

The medical moldings of the present invention may be, for example, parts for artificial dialyzers, parts for artificial livers, parts for artificial kidneys, parts for artificial hearts and lungs, parts for injectors and serum separators.

The physiological solution is, for example, an artificial dialyzing solution, physiological saline water, Ringer's solution or a maintaining solution.

Examples of γ-ray resistant materials comprising aromatic polycarbonate resins are:

(1) Those obtained by blending a polymer prepared by a phosgene process of reacting a dihydroxydiaryl compound with phosgene, or an ester exchanging process of reacting a dihydroxydiaryl compound with a carbonate such as diphenyl carbonate, with an organic bromine-containing compound such that the γ-ray resistant material contains from 0.1 to 10% by weight of bromine atoms as a whole.

Examples of typical polymers are those using 2,2-bis(4-hydroxyphenyl) propane (bisphenol A) as the dihydroxydiaryl compound comonomer.

(2) Those obtained by a phosgene process of reacting a dihydroxydiaryl compound and a brominated dihydroxydiaryl compound with phosgene, or an ester exchanging process of reacting a dihydroxydiaryl compound and a brominated dihydroxydiaryl compound with a carbonate such as diphenyl carbonate such that the γ-ray resistant material contains from 0.1 to 10.0% by weight of bromine atoms as a whole.

Examples of the copolymer are those using 2,2-bis(4-hydroxyphenyl)propane (bisphenol A) and bromine derivative thereof (brominated bisphenol A) as the monomers.

The process for producing a copolymer is known to those skilled in the art. There can be used, for example, a method as described in US-A-3,334,154 and JP-B-60-21691 (1985).

(3) Those obtained by blending a copolymer prepared by a phosgene process of reacting a dihydroxydiaryl compound and a brominated dihydroxydiaryl compound with phosgene, or an ester exchanging process of reacting a dihydroxydiaryl compound and a brominated dihydroxydiaryl compound with a carbonate such as diphenyl carbonate (the bromine content is not restricted to less than 10 wt%), with a polymer prepared by a phosgene process of reacting a dihydroxydiaryl compound with phosgene, or an ester exchanging process of reacting a dihydroxydiaryl compound with a carbonate such as diphenyl carbonate, such that the γ-ray resistant material contains from 0.1 to 10 wt% of bromine atoms as a whole.

As the dihydroxyaryl compound there can be mentioned, for example, bis(hydroxyaryl) alkanes such as 2,2-bis(4-hydroxyphenyl) propane (bisphenol A), bis(4-hydroxyphenyl) methane, 1,1-bis(4-hydroxyphenyl) ethane, 2,2-bis(4-hydroxyphenyl) butane, 2,2-bis(4-hydroxyphenyl) octane, bis(4-hydroxyphenyl) phenyl methane, 2,2-bis(4-hydroxyphenyl-3-methylphenyl) propane, 1,1-bis(4-hydroxy-3-tertiarybutylphenyl) propane, 2,2-bis(4-hydroxy-3-bromophenyl) propane, 2,2-bis(4-hydroxy-3,5-dibromophenyl) propane and 2,2-bis(4-hydroxy-3,5-dichlorophenyl) propane; bis(hydroxyaryl) cycloalkanes such as 1,1-bis(4-hydroxyphenyl) cyclopentane and 1,1-bis(4-hydroxyphenyl) cyclohexane; dihydroxydiaryl ethers such as 4,4'-dihydroxyphenyl ether and 4,4'-dihydroxy-3,3'-diethyldiphenyl ether; dihydroxydiaryl sulfides such as 4,4'-dihydroxydiphenyl sulfide and 4,4'-dihydroxy-3,3'-dimethyldiphenyl sulfide; dihydroxydiaryl sulfoxides such as 4,4'-dihydroxydiphenyl sulfoxide and 4,4'-dihydroxy-3,3'-dimethyldiphenyl sulfoxide; and dihydroxydiaryl sulfones such as 4,4'-dihydroxydiphenyl sulfone, and 4,4'-dihydroxy-3,3'-dimethyldiphenyl sulfone.

These compounds may be used singly or as a mixture of two or more. In addition, at least one of, for example, piperadine, dipiperidyl hydroquinone, resorcine and 4,4'-dihydroxydiphenyl may be mixed therewith.

The brominated dihydroxydiaryl compound is a compound prepared by partially or entirely substituting a hydrogen atom(s) on the phenyl group in a dihydroxydiaryl compound with bromine. There can be mentioned, for example:

2,2-bis(4-hydroxy-3,5-dibromophenyl) propane (tetrabromobisphenol A),
2,2-bis(4-hydroxy-3-bromophenyl) propane, and
2,2-bis(4-hydroxy-2,3,5,6-tetrabromophenyl) propane.

The organic bromine compound contains bromine atoms. As such a compound, there can be mentioned, for example, brominated benzenes such as hexabromobenzene and pentabromotoluene; brominated diphenyls such as tetrabromodiphenyl, decabromodiphenyl, tetrabromodiphenyl ether, decabromodiphenyl ether and decabromodiphenyl sulfone; brominated bisphenol A or the derivatives thereof such as 2,2-bis(4-hydroxy-3,5-dibromophenyl) propane (tetrabromobisphenol A), 2,2-bis(4-glycidoxy-3,5-dibromophenyl) propane or low polymers thereof (brominated bisphenol A-type epoxy resin), 2,2-bis(4-hydroxyethoxy-3,5-dibromophenyl) propane, 2,2-bis(4-acetoxy-3,5-dibromophenyl) propane and a polycarbonate oligomer of tetrabromobisphenol A; tetrabromophthalic acid anhydride; tribromophenyl condensates; polypentabromobenzyl acrylate; brominated polystyrene; and condensates of tetrabromobisphenol A, cyanuric acid and tribromophenol. A polycarbonate oligomer prepared from brominated bisphenol A such as a polycarbonate oligomer of tetrabromobisphenol A is particularly preferred.

The bromine atom content of the γ-ray resistant material of the present invention is from 0.1 to 10% by weight, preferably, 0.5 to 8% by weight.

If the amount of the bromine atoms is less than 0.1% by weight, the effect of improving the γ-ray resistance is poor, whereas the mechanical property of the composition is deteriorated or the heat-stability on melt-molding is poor if it is more than 10% by weight.

If polypropylene glycol is blended with the aromatic polycarbonate resins the $\gamma$-ray resistance can further be improved.

The molecular weight of polypropylene glycol used in the present invention is preferably not greater than 8,000, more preferably 1,000 to 8,000. If the molecular weight exceeds 8,000, the transparency tends to be deteriorated. The blending amount of polypropylene glycol is from 0.1 to 5% by weight, preferably from 0.5 to 2% by weight, based on the weight of the $\gamma$-ray resistant material. If it is less than 0.1% by weight, a sufficient effect cannot be developed. On the other hand, if it exceeds 5% by weight, it tends to deteriorate the transparency and mechanical properties.

As a method of blending the organic bromine-containing compound or polypropylene glycol, various means well-known to those skilled in the art can be used at any stage before the molding of the final molded product.

In the $\gamma$-ray resistant materials other resins, for example, polyethylene terephthalate, polybutylene terephthalate, polyester polycarbonate, polyarylate, polycondensate of cyclohexane dimethanol and terephthalic acid and/or isophthalic acid, or a copolymer of them with polyethylene terephthalate may be contained in a small amount such that the effect of the present invention is not impaired.

The $\gamma$-ray resistant materials may also contain various well-known additives, for example, a hydrolysis inhibitor such as an epoxy compound, lubricant such as paraffin wax and fatty acid ester, anti-oxidant such as a hindered phenol, phosphoric acid ester or phosphorous acid ester, weather proofness improver such as triazine compound or colourant such as a pigment or dye.

Medical moldings, such as medical instruments, of the present invention are obtained by molding the $\gamma$-ray resistant materials by methods well known to those skilled in the art, with no particular restriction. For example, injection molding, extrusion molding, blow molding or press molding can be used.

The medical moldings of the present invention can be subjected to irradiation by $\gamma$-rays for sterilization. They show little yellowing even after undergoing such sterilizing treatment.

The sterilized medical molding of the present invention is obtained by irradiating $\gamma$-rays to a medical molding comprising a $\gamma$-ray resistant material comprising an aromatic polycarbonate resin, which $\gamma$-ray resistant material contains from 0.1 to 10% by weight of bromine atoms.

The irradiation dose of $\gamma$-rays is usually 2 to 5 Mrad. The irradiating time and strength of $\gamma$-rays may be adjusted so that the dose is within the above-mentioned dose range.

It is possible to produce medical moldings which are fully sterilized until they are used for medical treatment, and which have little yellowing even having undergone the sterilizing treatment, by the above method. It is thus possible to provide medical parts which are highly resistant to ionizing radiation.

The present invention is now further explained specifically in the following Examples.

Examples 1 - 7 and Comparative Examples 1 - 2:

As the aromatic polycarbonate resin, a bisphenol A polycarbonate resin having a viscosity average molecular weight of 22,000 (NOVAREX 7022A: trade name of Mitsubishi Kasei Corp.) was blended with a polycarbonate oligomer of tetrabromobisphenol A (BC-58, trade name of Great Lakes Co.), decabromodiphenyl ether (DE-83P : trade name of Great Lakes Co.) and polypropylene glycol having a molecular weight of 4,500 (E-4500 : trade name of Dow Chemical Co.) in a composition as shown in Table 1 and pelletized by an extruder having a bent with a screw diameter of 40 mm$\phi$ at a resin temperature of from 270 to 290 °C. After drying the pellets, test specimens (each of 50 mm$\phi$ x 3 mm thickness) were prepared by using an injection molding machine with a mold clamping pressure of 75 ton at a resin temperature range of from 280 to 300 °C.

The yellowness index of the resultant test specimen was measured in accordance with JIS K-7103 and, furthermore, the test specimen was subjected to sterilization by the irradiation of $\gamma$-rays at 2.5 Mrad in an air and oxygen-free atomosphere with the aid of the Nippon Isotope Association. Then yellowness index was again measured after two days. The results are shown in Table 1. The oxygen-free atmosphere was obtained by tightly sealing the specimen together with an oxygen remover (Ageless: trade name of Mitsubishi Gas Chemical Company, Inc.).

Table 1

| | Composition (wt%) | | | | Yellowness index before γ-ray irradiation | Yellowness index after γ-ray irradiation | | | |
| --- | --- | --- | --- | --- | --- | --- | --- | --- | --- |
| | Aromatic polycarbonate resins | Tetrabromobisphenol A oligomer | Decabromodiphenyl ether | Polypropylene glycol | | Air | | Oxygen-free atomosphere | |
| | | | | | | Measured value | Amount changed | Measured value | Amount changed |
| Example 1 | 98.3 | 1.7(1.0) | – | – | 2.7 | 11.2 | 8.5 | 9.6 | 6.9 |
| Example 2 | 97.3 | 1.7(1.0) | – | 1.0 | 3.0 | 9.5 | 6.5 | 8.8 | 5.8 |
| Example 3 | 94.8 | 5.2(3.0) | – | – | 3.2 | 8.5 | 5.3 | 6.7 | 3.5 |
| Example 4 | 94.3 | 5.2(3.0) | – | 0.5 | 3.4 | 7.9 | 4.5 | 7.0 | 3.6 |
| Example 5 | 93.8 | 5.2(3.0) | – | 1.0 | 3.4 | 5.2 | 1.8 | 4.6 | 1.2 |
| Example 6 | 87.9 | 12.1(7.0) | – | – | 3.7 | 5.6 | 1.9 | 4.8 | 1.1 |
| Example 7 | 96.4 | – | 3.6(3.0) | – | 3.3 | 11.6 | 8.3 | 10.8 | 7.5 |
| Comparative Example 1 | 100 | – | – | – | 2.5 | 30.3 | 27.8 | 54.5 | 52.0 |
| Comparative Example 2 | 99.0 | – | – | 1.0 | 2.9 | 14.2 | 11.3 | 27.9 | 25.0 |

(Note) Numerical value in ( ) represents the content (wt%) as **bromine** atom.

Reference Examples 1 - 3:

Copolymerized polycarbonate resins comprising bisphenol A and tetrabromobisphenol A was obtained by a method as described in JP-B-60-21691 (1985). The properties of the resins are:

Reference Example 1 :

| Molecular weight | 21,500 |
|---|---|
| Bromine-content | 0.5 wt% |

Reference Example 2 :

| Molecular weight | 21,900 |
|---|---|
| Bromine-content | 2.5 wt% |

Reference Example 3 :

| Molecular weight | 21,300 |
|---|---|
| Bromine-content | 7.5 wt% |

Reference Example 4:

Commercially available bisphenol A polycarbonate resin having a molecular weight of 22,000 not containing halogen (NOVAREX 7022A, trade mark of Mitsubishi Kasei Corp.)

Examples 8 - 13:

The brominated aromatic polycarbonate resins in Reference Examples 1 to 3, alone or blended with commercially available polypropylene glycol having a molecular weight of 4,500 (E-4500 : manufactured by Dow Chemical Co.) each in the composition as shown in Table 2, were pelletized using an extruder with a bent with screw diameter of 40 mm$\phi$ at a resin temperature of from 270 to 290°C. After drying the pellets, test specimens (each of 50 mm$\phi$ x 3 mm thickness) were prepared using an injection molding machine with a mold clamping pressure of 75 ton at a resin temperature range of from 280 to 300°C.

The yellowness index of the thus obtained test specimens was measured in accordance with JIS K-7103 and, furthermore, the specimens were sterilized by the irradiation of $\gamma$-rays at 2.5 Mrad in air with the aid of the Nippon Isotope Association. Then the yellowness index was measured again after two days. The results obtained are shown in Table 2.

Comparative Examples 3 - 4:

The same procedures as those in Examples 8 - 13 were conducted except for using the polycarbonate resin not containing halogen in Reference Example 4.

Table 2

| | Aromatic polycarbonate resin | Polypropylene glycol (part by weight) | Yellowness index | | |
|---|---|---|---|---|---|
| | | | Before γ-ray irradiation | After γ-ray irradiation | Amount changed |
| Example 8 | Reference Example 1 | – | 2.8 | 8.3 | 5.5 |
| Example 9 | Reference Example 1 | 1.0 | 3.1 | 7.4 | 4.3 |
| Example 10 | Reference Example 2 | – | 3.4 | 7.1 | 3.7 |
| Example 11 | Reference Example 2 | 0.5 | 3.6 | 6.3 | 2.9 |
| Example 12 | Reference Example 2 | 1.0 | 3.5 | 5.9 | 2.4 |
| Example 13 | Reference Example 3 | – | 3.8 | 5.8 | 2.0 |
| Comparative Example 3 | Reference Example 4 | – | 2.6 | 32.4 | 29.8 |
| Comparative Example 4 | Reference Example 4 | 1.0 | 3.0 | 15.1 | 12.1 |

Example 14:

100 parts by weight of the copolymerized polycarbonate resin of Reference Example 1 was blended with 1.0 part by weight of polypropylene glycol (E-4500; trade name of Dow Chemical Co.) and pelletized using an extruder with a bent with screw diameter of 40 mmφ at a resin temperature range of from 270 to 290°C. After drying the pellets, ten dialyzer outer-cylinders each having an inner diameter of 40 mmφ, an outer diameter of 45 mmφ and length of 225 mm disposed with an introduction/discharge port of 12 mmφ each at the upper end and the lower end were molded using an injection molding machine with a mold clamping pressure of 160 ton at a resin temperature range of from 290 to 310°C. Pieces of 30 mm x 30 mm were cut out from the middle portion (2 mm thickness) of five of the ten outer-cylinders, and the yellowness index was measured in accordance with JIS K-7103. The remaining five outer-cylinders were sterilized under irradiation of Co-60 γ-rays at 2.5 Mrad in air with the aid of the Nippon Isotope Association. Then, two days after, specimens of 30 mm x 30 mm were cut out from the middle portions thereof in the same manner as described above and the yellowness index was measured. The results obtained are shown in Table 3.

Comparative Example 5:

The procedures were conducted in the same as those in Example 14 except for using the polycarbonate not containing halogen in Reference Example 4 and not blending polypropylene glycol. The results are shown in Table 3.

Table 3

| | | Yellowness index | | | | | |
|---|---|---|---|---|---|---|---|
| | | Each value of specimens | | | | | Average value |
| Example 14 | Before γ-ray irradiation | 3.0 | 2.9 | 3.2 | 2.6 | 2.7 | 2.9 |
| | After γ-ray irradiation | 6.5 | 6.1 | 6.4 | 6.1 | 6.2 | 6.3 |
| Comparative Example 5 | Before γ-ray irradiation | 2.4 | 2.0 | 2.3 | 2.3 | 2.1 | 2.2 |
| | After γ-ray irradiation | 22.5 | 23.3 | 21.2 | 22.0 | 22.9 | 22.4 |

**Claims**

1. A medical molding suitable for contacting with blood or a physiological solution, comprising a γ-ray resistant material comprising an aromatic polycarbonate resin, which γ-ray resistant material contains from 0.1 to 10% by weight of bromine atoms.

2. A molding according to claim 1, wherein the γ-ray resistant material comprises a brominated aromatic polycarbonate copolymer.

3. A molding according to claim 1, wherein the γ-ray resistant material comprises an aromatic polycarbonate polymer and an organic bromine-containing compound.

4. A molding according to claim 1, wherein the γ-ray resistant material comprises an aromatic polycarbonate polymer and a brominated aromatic polycarbonate copolymer.

5. A molding according to any one of the preceding claims, wherein the γ-ray resistant material comprises from 0.1 to 5% by weight of polypropylene glycol.

6. A molding according to any one of the preceding claims, which is part of an artificial dialyzer.

7. A molding according to any one of claims 1 to 6, which is a part for an artificial liver, artificial heart, artificial lung, an injector or a serum separator.

8. A method of sterilizing a medical molding which comprises irradiating a molding as defined in any one of the preceding claims with γ-rays.

9. A γ-ray resistant material as defined in any one of claims 1 to 5.

10. A medical molding as defined in any one of claims 1 to 7 which is sterile.

**Patentansprüche**

1. Medizinischer Formkörper, geeignet zum Kontaktieren mit Blut oder einer physiologischen Lösung, umfassend ein gammastrahlenresistentes Material, das ein aromatisches Polycarbonatharz enthält, wobei das gammastrahlenresistente Material von 0,1 bis 10 Gew.% Bromatome enthält.

2. Formkörper nach Anspruch 1,
dadurch **gekennzeichnet,** daß
das gammastrahlenresistente Material ein bromiertes aromatisches Polycarbonatcopolymer umfaßt.

3. Formkörper nach Anspruch 1,
dadurch **gekennzeichnet,** daß
das gammastrahlenresistente Material ein aromatisches Polycarbonatpolymer und eine organische bromhaltige Verbindung umfaßt.

4. Formkörper nach Anspruch 1,
dadurch **gekennzeichnet,** daß
das gammastrahlenresistente Material ein aromatisches Polycarbonatpolymer und ein bromiertes aromatisches Polycarbonatcopolymer umfaßt.

5. Formkörper nach einem der vorhergehenden Ansprüche,
dadurch **gekennzeichnet,** daß
das gammastrahlenresistente Material von 0,1 bis 5 Gew.% Polypropylenglycol umfaßt.

6. Formkörper nach einem der vorhergehenden Ansprüche,
dadurch **gekennzeichnet,** daß
er ein Teil eines künstlichen Dialysators ist.

7. Formkörper nach einem der Ansprüche 1 bis 6,
dadurch **gekennzeichnet,** daß
er ein Teil für eine künstliche Leber, künstliches Herz, künstliche Lunge, einen Injektor oder einen Serumtrenner ist.

8. Verfahren zum Sterilisieren eines medizinischen Formkörpers, umfassend Bestrahlen eines Formkörpers nach einem der vorhergehenden Ansprüche mit Gamma-Strahlen.

9. Gammastrahlenresistentes Material wie in einem der Ansprüche 1 bis 5 definiert.

10. Medizinische Formkörper nach einem der Ansprüche 1 bis 7, dadurch **gekennzeichnet,** daß er steril ist.

**Revendications**

1. Pièce médicale moulée convenant à un contact avec du sang ou une composition physiologique, comprenant une matière résistant aux rayons $\gamma$, contenant une résine de polycarbonate aromatique, ladite matière résistant aux rayons $\gamma$ contenant 0,1 à 10 % en poids d'atomes de brome.

2. Pièce moulée selon la revendication 1, dans laquelle la matière résistant aux rayons $\gamma$ est constituée d'un copolymère de polycarbonate aromatique bromé.

3. Pièce moulée selon la revendication 1, dans laquelle la matière résistant aux rayons $\gamma$ est constituée d'un polymère de polycarbonate aromatique et d'un composé organique contenant du brome.

4. Pièce moulée selon la revendication 1, dans laquelle la matière résistant aux rayons $\gamma$ est constituée d'un polymère d'un polycarbonate aromatique et d'un copolymère de polycarbonate aromatique bromé.

5. Pièce moulée selon l'une quelconque des revendications précédentes, dans laquelle la matière résistant aux rayons $\gamma$ comprend 0,1 à 5 % en poids de polypropylène glycol.

6. Pièce moulée selon l'une quelconque des revendications précédentes, qui fait partie d'un dialyseur artificiel.

7. Pièce moulée selon l'une quelconque des revendications 1 à 6, qui fait partie d'un foie, d'un coeur et d'un poumon artificiels, d'un injecteur ou d'un séparateur de sérum.

8. Procédé de stérilisation d'une pièce médicale moulée qui consiste à irradier une pièce moulée selon l'une quelconque des revendications précédentes par des rayons $\gamma$.

9. Matière résistant aux rayons $\gamma$ selon l'une quelconque des revendications 1 à 5.

10. Pièce médicale moulée stérile selon l'une quelconque des revendications 1 à 7.